Europäisches Patentamt

European Patent Office

Office européen des brevets

(11) Veröffentlichungsnummer: **0 351 864**

**A1**

## (12) EUROPÄISCHE PATENTANMELDUNG

(21) Anmeldenummer: **89113408.2**

(22) Anmeldetag: **21.07.89**

(51) Int. Cl.⁴: **A61M 25/00**

(30) Priorität: **21.07.88 DE 8809319 U**

(43) Veröffentlichungstag der Anmeldung:
**24.01.90 Patentblatt 90/04**

(84) Benannte Vertragsstaaten:
**AT BE CH DE ES FR GB GR IT LI LU NL SE**

(71) Anmelder: **MEDINORM
AKTIENGESELLSCHAFT
MEDIZINTECHNISCHE PRODUKTE
Stiftstrasse 4
D-6607 Quierschied(DE)**

(72) Erfinder: **Fell, Helmut Dr. rer. nat.,
Grumbachweg 28a
D-6607 St. Ingbert(DE)**

(74) Vertreter: **Müller, Hans
Lerchenstrasse 56
D-7100 Heilbronn(DE)**

(54) **Katheterventil.**

(57) Ein Katheter (10) mit einem an seinem Endbereich vorhandenen Ventil zeichnet sich dadurch aus, daß der Katheter (10) in seinem Endbereich (14) bis auf zumindest einen Schlitz (16) in seiner Wandung verschlossen ist, wobei der stirnseitige Verschluß aus seinem Wandbereich (15) des Katheters (10) gebildet ist, so daß der Katheter und das durch den zumindest einen Schlitz gebildete Ventil einstückig miteinander verbunden sind.

FIG.1

## TECHNISCHES GEBIET

Die Erfindung betrifft einen Katheter mit einem an seinem einen Endbereich vorhandenen Ventil.

Ein Katheter ist ein röhren- bzw. schlauchförmiges medizinisches Instrument, das zum Einführen in Körperhohlorgane (z. B. Vene, Arterie, Harnblase, Herz) verwendet wird. Durch den Katheter wird entweder in das betreffende Organ ein flüssiges Medikament ein- oder Flüssigkeit aus den Organ herausgeführt.

Damit sichergestellt ist, daß durch einen eingeführten Katheter nicht unbeabsichtigt Blut oder sonstige Flüssigkeit durch den Katheter hindurch rückwärts, nach außen entweichen kann, ist das körperseitige Ende, d. h. die vordere Spitze des Katheters mit einem Rückschlagventil versehen. Dieses Ventil öffnet sich nur dann, wenn durch den Katheter unter einem vorbestimmten Druck Flüssigkeit hindurchgedrückt wird. Diese Flüssigkeit kann dann durch das Ventil hindurch in die Vene, Arterie oder in das sonstige vorhandene hohle Körperorgan einströmen.

### STAND DER TECHNIK

Bei den üblicherweise verwendeten Kathetern ist das Rückschlagventil als separates Bauteil an dem einen Ende des schlauchartigen Katheters befestigt. Die Befestigung erfolgt in aller Regel über eine Klebverbindung. Da infolge der sehr geringen Dicke des Katheterschlauchs das Ventil nicht stirnseitig an der Schlauchwandung angeklebt werden kann, über greift das Ventil regelmäßig das stirnseitige Ende der Wandung. Dies hat einmal den Nachteil, daß der Katheterkopf im Durchmesser dicker ist als der übrige schlauchartige Körper des Katheters, wodurch das Einführen eines derartigen Katheters nur unter erschwerten Bedingungen möglich ist. Auch ist die Gefahr, daß durch den verdickten Kopfbereich die Innenfläche einer Arterie oder Vene verletzt wird, nicht unerheblich. Die Verwendung derartiger Katheter wird ferner dadurch beeinträchtigt, daß sich in nicht wenigen Fällen das Ventil vom Katheter löst, dá die Klebverbindung zwischen den beiden Bauteilen nicht dauerhaft genug ist.

### DARSTELLUNG DER ERFINDUNG

Ausgehend von diesem vorbekannten Stand der Technik liegt der Erfindung die Aufgabe zugrunde, einen Katheter mit einem an seinem einen Endbereich vorhandenen Ventil anzugeben, der einfach in der Herstellung und problemlos in seiner Anwendung ist.

Diese Erfindung ist durch die Merkmale des Hauptanspruchs gegeben. Sie zeichnet sich bei dem vorstehend genannten Katheter dementsprechend dadurch aus, daß der Katheter in seinem Endbereich bis auf zumindest einen Schlitz in seiner Wandung verschlossen ist, wobei der stirnseitige Verschluß aus einem Wandbereich des Katheters gebildet ist, so daß der Katheter und das durch den zumindest einen Schlitz gebildete Ventil einstückig miteinander verbunden sind.

Ein derartiger Katheter braucht an seinem ventilseitigen Endbereich nicht dicker zu sein als in seinem übrigen, schlauchartigen Bereich, so daß sein Einführen in verengte Venen oder dergleichen einfacher wird und darüber hinaus die Gefahr einer Verletzung der Venenwandung oder dergleichen verringert wird. Auch kann bei diesem Katheter absolut sichergestellt werden, daß sich das Ventil nicht mehr vom Katheterschlauch löst, weil ja Katheter und Ventil aus der Mantelfläche des Katheterschlauchs als einteiliges Bauteil vorhan den sind. Da neben diesen in der medizinischen Anwendung begründeten Vorteile auch die Herstellung eines solchen Katheters billiger wird, erweist sich der erfindungsgemäße Katheter als optimales neues medizinisches Instrument.

Der die Ventilöffnung bildende Schlitz kann entweder in dem Mantelbereich oder in dem Stirnflächenbereich des Katheter-Endbereiches vorhanden sein. Die Anordnung des Schlitzes in dem Mantelbereich hat insbesondere den Vorteil, daß die stirnseitige Mantelfläche vollständig verschlossen bleiben kann. Dadurch ist die Gefahr ausgeschlossen, daß beim Einführen des Katheters Festkörper in den Schlitzbereich eindringen und dadurch die Schließwirkung des Schlitzes beeinträchtigen könnten. Die Anordnung des Schlitzes in dem stirnseitigen Mantelbereich ermöglicht dagegen insbesondere ein sehr leichtes Einspritzen von flüssigen Medikamenten in eine Vene, Arterie oder dergleichen.

Der erfindungsgemäße Katheter kann sehr billig aus Polyurethan oder Weich-PVC hergestellt werden. Derartige thermoplastische Kunststoffe erlauben nämlich eine sehr einfache Herstellung des "Ventils"; diese könnte folgendermaßen aussehen:

Das schlauchartige Ende des Katheters wird in eine Silikongummilösung getaucht, um so die Innenseite der Katheterwandung mit diesem Silikongummi zu benetzen. Anschließend wird der Endbereich des Katheters durch Erwärmung erweicht und plattgedrückt, so daß die stirnseitigen Enden des Katheters in gegenseitige Anlage kommen. Das zwischen den Katheterwandungen vorhandene Silikongummi verhindert, daß die beiden Wandbereiche sich dabei miteinander bleibend verbinden. Bei diesem Plattdrücken wird der maximale Durchmesser des Katheterschlauches an seinem plattgedrückten stirnseitigen Ende größer als im übrigen Schlauchbereich. Durch schräges Kappen der gegenüberliegenden "Spitzen" wird die durch diese Spitzen mögliche Verletzungsgefahr einer Venen- oder Arterienwandung vermieden.

Während im letzteren Fall das stirnseitige Ende des Katheters den "Ventil "-Schlitz bereits aufweist, ist in den vorstehend genannten beiden Ausführungsformen der Schlitz - entweder in die Mantelfläche oder in die stirnseitige Fläche - nachträglich anzubringen, was ohne besondere Schwierigkeiten möglich ist.

Weitere Ausführungsformen und Vorteile der Erfindung sind den in den Ansprüchen weiterhin aufgeführten Merkmalen zu entnehmen.


## KURZE BESCHREIBUNG DER ZEICHNUNG


Die Erfindung wird im folgenden anhand der in der Zeichnung dargestellten Ausführungsbeispiele näher beschrieben und erläutert. Es zeigen:

Fig. 1 die schematische Ansicht des Endbereichs eines Katheters nach der Erfindung in einer ersten Ausführungsform,

Fig. 2 eine zweite Ausführungsform eines Katheters,

Fig. 3 eine weitere Ausführungsform des Katheters und

Fig. 4 einen Längsschnitt entlang Linie 4-4 der Fig. 3.


## WEGE ZUR AUSFÜHRUNG DER ERFINDUNG


Ein schlauchartiger Katheter 10 besitzt in seinem "normalen" Schlauchbereich eine Mantelfläche 12. An seinem - in der Zeichnung rechten - Endbereich 14 ist er thermoplastisch so verformt, daß dieser Endbereich 14 ebenfalls durch einen Mantelflächenbereich 15 verschlossen ist. Die Bereiche der Mantelfläche 12 und 15 sind thermoplastisch miteinander verschweißt, so daß der Katheter in seinem Endbereich 14 ein stirnseitig verschlossener hohler Körper ist. Der Katheter ist stark vergrößert dargestellt. Sein wahrer Durchmesser liegt in der Größenordnung von 1 mm.

In die Mantelfläche 12 des Endbereichs 14 ist in Längsrichtung L ein Schlitz 16 eingeschnitten. Beim Einführen von Flüssigkeiten in Richtung 18 in den Katheter 10 hinein, weitet sich bei einem bestimmten Überdruck der Schlitz 16 aus, so daß die Flüssigkeit durch den Schlitz 16 in eine nicht dargestellte Vene, Arterie oder dergleichen heraustreten kann.

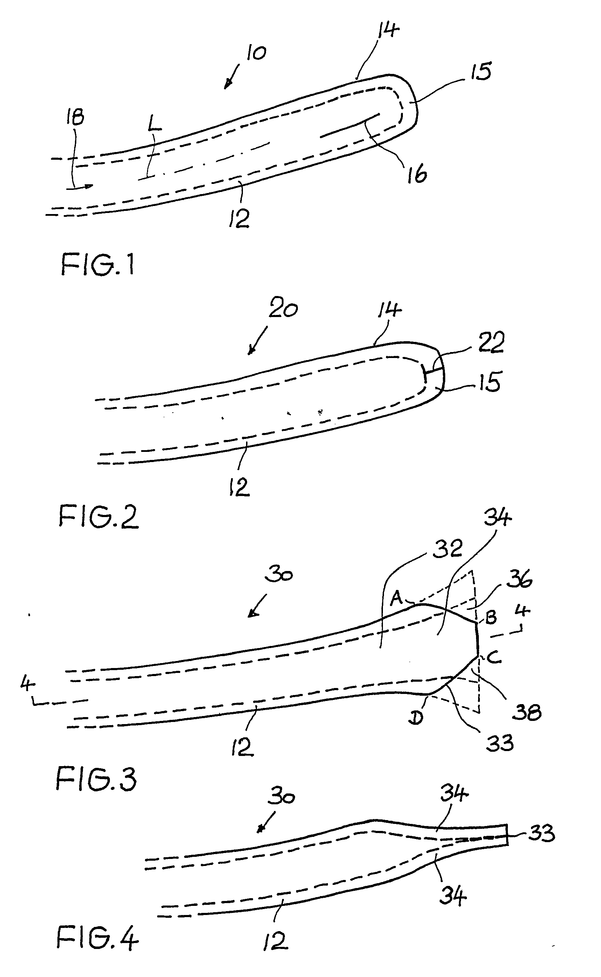

Der in Fig. 2 dargestellte Katheter 20 unterscheidet sich von dem Katheter 10 dadurch, daß ein Schlitz 22 in seinem stirnseitigen Mantelflächenbereich 15 und nicht in seiner Mantelfläche 12 vorhanden ist. Auch dieser Schlitz 22 ist nachträglich in den stirnseitigen Mantelflächenbereich 15 eingeschnitten worden.

Bei dem Katheter 30 (Fig. 3) ist nachträglich kein Schlitz entsprechend der Schlitze 16, 22 der Figuren 1 und 2 angebracht worden. Der Endbereich 32 dieses Katheters 30 ist thermoplastisch zu der in Fig. 4 dargestellten Form verformt worden. Durch Benetzen der Innenfläche des Mantels 12 ist bei dieser thermoplastischen Verformung erreicht worden, daß sich die im Endbereich 32 vorhandene Mantelfläche 34 nicht miteinander verschweißt. Die bei dieser Verformung vorhandenen - gestrichelt dargestellten - beiden Eckbereiche 36, 38 sind nachträglich abgetrennt worden, so daß der Endbereich 32 eine Umrißlinie 33 erhält, die längs der Punkte A, B, C, D verläuft. In den Bereichen A und D weist die Schnittlinie eine Ausrundung auf, um zu vermeiden, daß die ansonsten vorhandenen Ecken beim Einführen des Katheters 30 in Körperorgane zu Verletzungen führen könnten. Bei dem Katheter 30 ist der die Ventilwirkung bewirkende Schlitz damit längs der Umrißlinie A, B, C, D vorhanden. Der Schlitz reicht dabei nicht ganz bis zu den Endpunkten A, B hin, da dort noch die stirnseitig verschlossenen Wandbereiche der Mantelfläche 34 vorhanden sind.

Selbstverständlich ist es möglich, nicht nur einen Schlitz 16, 22 sondern mehrere derselben anzuordnen, die dann sowohl in der stirnseitigen Mantelfläche 15 (Fig. 2) als auch in der Mantelfläche 12 (Fig. 1) vorhanden sein können. Bei dem Katheter 30 ist es darüber hinaus möglich, auch in seiner Mantelfläche 12 einen dem Schlitz 16 vergleichbaren Schlitz zusätzlich anzuordnen. Die Anzahl und Auswahl der Schlitze hängt dabei von der Rückstellkraft des vorhandenen Kunststoffmaterials und von der Flüssigkeitsmenge ab, die durch den jeweiligen Katheter hindurchgedrückt werden soll.


## Ansprüche


01) Katheter mit einem an seinem einen Endbereich vorhandenen Ventil,
**dadurch gekennzeichnet,** daß der Katheter (10, 20, 30) in seinem Endbereich (14, 32) bis auf zumindest einen Schlitz (16, 22, 33) in seiner Wandung verschlossen ist, wobei der stirnseitige Verschluß aus einem Wandbereich (15, 34) des Katheters (10, 20, 30) gebildet ist, so daß der Katheter und das durch den zumindest einen Schlitz gebildete Ventil einstückig miteinander verbunden sind.

02) Katheter nach Anspruch 1,
**dadurch gekennzeichnet,** daß der Schlitz (16) in dem Mantelbereich (12) des Endbereiches vorhanden ist.

03) Katheter nach Anspruch 1 oder 2,
**dadurch gekennzeichnet,** daß der Schlitz (22) in dem Stirnflächenbereich (15) des Endbereiches vorhanden ist.

04) Katheter nach Anspruch 1,
**dadurch gekennzeichnet,** daß der Katheter (10, 20, 30) samt Ventil aus einem thermoplastischen Kunststoff, insbesondere aus Polyurethan oder Weich-PVC besteht, und der verschlossene Endbereich (14) einen thermoplastisch verschweißten Wandbereich aufweist.

FIG.1

FIG.2

FIG.3

FIG.4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | EP-A-0 125 844 (CATHETER TECHNOLOGY CORP.)<br>* Figuren 1-4 *<br>--- | 1,2,4 | A 61 M 25/00 |
| X | EP-A-0 250 981 (BECTON, DICKINSON & CO.)<br>* Figuren 1-7 *<br>--- | 1,2,4 | |
| A | DE-U-8 710 340 (CYTOMED)<br>* Figuren 1,2 *<br>--- | 1,3 | |
| A | DE-U-8 503 774 (CYTOMED)<br>* Figur 2 *<br>--- | 1,3 | |
| A | US-A-4 578 061 (LEMELSON)<br>* Figuren 2,3; Spalte 2, Zeile 65 -<br>Spalte 3, Zeile 3 *<br>----- | 1,3 | |

RECHERCHIERTE SACHGEBIETE (Int. Cl.5)

A 61 M

Der vorliegende Recherchenbericht wurde für alle Patentansprüche erstellt

| Recherchenort | Abschlußdatum der Recherche | Prüfer |
|---|---|---|
| DEN HAAG | 25-08-1989 | RAKOWICZ,J.M. |

KATEGORIE DER GENANNTEN DOKUMENTE

X : von besonderer Bedeutung allein betrachtet
Y : von besonderer Bedeutung in Verbindung mit einer
  anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
  nach dem Anmeldedatum veröffentlicht worden ist
D : in der Anmeldung angeführtes Dokument
L : aus andern Gründen angeführtes Dokument
........................................................
& : Mitglied der gleichen Patentfamilie, übereinstimmendes
  Dokument